(19) Europäisches Patentamt
European Patent Office
Office européen des brevets

(11) Veröffentlichungsnummer: **0 143 911**
**B1**

(12) # EUROPÄISCHE PATENTSCHRIFT

(45) Veröffentlichungstag der Patentschrift: **28.01.87**

(51) Int. Cl.⁴: **C 07 C 69/40**, C 07 C 67/38

(21) Anmeldenummer: **84110452.4**

(22) Anmeldetag: **03.09.84**

(54) **Verfahren zur Herstellung von Bernsteinsäurediestern.**

(30) Priorität: **06.09.83 DE 3332018**

(43) Veröffentlichungstag der Anmeldung: **12.06.85 Patentblatt 85/24**

(45) Bekanntmachung des Hinweises auf die Patenterteilung: **28.01.87 Patentblatt 87/5**

(84) Benannte Vertragsstaaten: **BE DE FR GB IT NL**

(56) Entgegenhaltungen:
**FR - A - 2 526 421**

**CHEMICAL ABSTRACTS, Band 70, Nr. 19, 12. Mai 1969 Columbus, Ohio, USA A. MATSUDA, "Cobalt carbonyl-catalyzed hydroesterification of methyl acrylate with carbon monoxide and methanol" Seite 253, Spalte 1, Zusammenfassung-Nr. 86978e**
**PATENT ABSTRACTS OF JAPAN, unexamined applications, C Field, Band 7, nr. 167, 22. Juli 1983 THE PATENT OFFICE JAPANESE GOVERNMENT Seite 1 C 177**

(73) Patentinhaber: **BASF Aktiengesellschaft, Carl-Bosch-Strasse 38, D-6700 Ludwigshafen (DE)**

(72) Erfinder: **Reuvers, Johannes G. Dr., Rathausstrasse 97, D-6806 Viernheim (DE)**
Erfinder: **Richter, Wolfgang, Dr., Osloer Weg 19, D-6700 Ludwigshafen (DE)**
Erfinder: **Kummer, Rudolf, Dr., Kreuzstrasse 6, D-6710 Frankenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 143 911 B1

**Beschreibung**

Die vorliegende Erfindung betrifft ein verbessertes Verfahren zur Herstellung von Bernsteinsäurediestern durch Carbonylierung von Acrylsäureestern in Gegenwart von Alkoholen mittels Cobaltcarbonyl-Komplexen als Katalysatoren und heterocyclischen Stickstoffbasen als Promotoren bei 80-200° C und unter einem Kohlenmonoxiddruck von 60-300 bar sowie in Gegenwart von mindestens 20 Gew.-%, bezogen auf die Menge aller flüssigen bestandteile des Reaktionsgemisches, einer inerten Flüssigkeit.

Die Carbonylierung von olefinisch ungesättigten Verbindungen unter den vorgenannten Bedingungen ist Gegenstand zahlreicher Untersuchungen und demgemäss allgemein bekannt.

Aus der FR-A-2 526 421 ist es speziell bekannt, hierbei in bestimmter Weise substituierte aromatische Kohlenwasserstoffe als Lösungsmittel zu verwenden, jedoch sind die Ausbeuten an linearen Carbonylierungsprodukten linearer olefinischer Verbindungen wie Penten- und Hexensäurenestern mit höchstens rd. 80% unbefriedigend.

Ferner ist es aus der Arbeit von A. Matsuda in Bull. Chem. Soc. Japan, Vol. 42, 1969, Seiten 571-72, speziell bekannt, Methylacrylat und Methanol zum Dimethylsuccinat umzusetzen:

$$CH_2{=}CH{-}CO{-}O{-}Me \xrightarrow[\text{Pyridin, MeOH}]{\text{CO; Co}} Me{-}O{-}CO{-}CH_2{-}CH_2{-}CO{-}O{-}Me$$

Zwar setzt sich das Methylacrylat hierbei vollständig um, jedoch werden nur Dimethylsuccinat-Ausbeuten von höchstens 82% erzielt. Der Rest entfällt grösstenteils auf nicht näher untersuchte Nebenprodukte sowie auf geringere Anteile an den Dimethylestern der Methylmalonsäure (dem isomeren Carbonylierungsprodukt des Acrylesters) und der 3-Ketopimelinsäure.

Da nach den vorbekannten Verfahren somit praktisch ein Fünftel der Einsatzstoffe wie Methylacrylat verloren gehen, kommen sie für technische Zwecke nicht in Betracht.

Der Erfindung lag daher die Aufgabe zugrunde, die Carbonylierung von Acrylsäureestern mit Kohlenmonoxid und Alkoholen so auszugestalten, dass man die Bernsteinsäurediester auf einfache Weise in zufriedenstellenden Ausbeuten erhält.

Demgemäss wurde ein verbessertes Verfahren zur Herstellung von Bernsteinsäurediestern durch Carbonylierung von Acrylsäureestern in Gegenwart von Alkoholen mittels Cobaltcarbonyl-Komplexen als Katalysatoren und heterocyclischen Stickstoffbasen als Promotoren bei 80-200° C und unter einem Kohlenmonoxiddruck von 60-300 bar sowie in Gegenwart von mindestens 20 Gew.%, bezogen auf die Menge aller flüssigen Bestandteile des Reaktionsgemisches, einer inerten Flüssigkeit gefunden, welches dadurch gekennzeichnet ist, dass man hierbei die Konzentration des Acrylsäureesters während des überwiegenden Teils der Reaktionsdauer unter 15 Gew.% hält, ebenfalls bezogen auf die Menge aller flüssigen Bestandteile.

Als inerte Flüssigkeiten sind wie üblich solche zu verstehen, die sich unter den Reaktionsbedingungen nicht oder nicht nennenswert verändern. Vorzugsweise verwendet man solche inerten Flüssigkeiten, die mit allen anderen flüssigen Bestandteilen des Reaktionsgemisches unter den Reaktionsbedingungen eine homogene flüssige Phase bilden, so dass die inerten Flüssigkeiten die Funktion eines Lösungsmittels haben. Da dies der Normalfall ist, werden die inerten Flüssigkeiten im folgenden als Lösungsmittel bezeichnet.

Als derartige Lösungsmittel eignen sich vornehmlich aromatische Kohlenwasserstoffe wie Benzol, Toluol oder die Xylole sowie daneben auch $C_6$-$C_{12}$-Alkane wie n-Heptan und n-Octan und Cycloalkane wie Cyclohexan. Mit besonderem Vorteil verwendet man indes den bei der Reaktion entstehenden Bernsteinsäurediester als Lösungsmittel.

Im Hinblick auf den Verfahrenserfolg ist die Konzentration des Lösungsmittels entsprechend den bisherigen Beobachtungen nach oben nicht begrenzt. Aus technisch-wirtschaftlichen Gründen empfiehlt sich jedoch im allgemeinen ein Konzentrationsbereich von 20-80, vorzugsweise 40-60 Gew.%.

Erfindungsgemäss ist die Konzentration des eigesetzten Acrylsäureesters während des überwiegenden Teils der Reaktionsdauer unter 15 Gew.%, bezogen auf die Gesamtmenge aller flüssigen Bestandteile, zu halten. Dies bedeutet einerseits, dass man bestrebt sein wird, diese Bedingung während der gesamten Reaktionsdauer einzuhalten, andererseits aber, dass gelegentliche Überschreitungen dieser Konzentration nicht von nennenswertem Nachteil sind.

Vorzugsweise hält man die Konzentration des Acrylsäureesters unter 5 Gew.%, lässt sie jedoch aus technisch-wirtschaftlichen Gründen nicht unter 1 Gew.% absinken.

Da die Reaktion exotherm ist und die freigesetzte Wärmemenge somit ein Mass für den umgesetzten Acrylsäureester darstellt, lässt sich die der Einhaltung einer bestimmten Konzentration entsprechende Zulaufgeschwindigkeit des Acrylsäureesters unschwer bestimmen. Eine andere Möglichkeit zur Steuerung der Acrylsäureester-Konzentration basiert auf regelmässigen GC-Analysen des Reaktionsgemisches. Ist die Zulaufgeschwindigkeit des Acrylsäureesters auf diese oder ähnliche Weise einmal bestimmt worden, bedarf es für weitere Reaktionsansätze oder für den kontinuierlichen Betrieb keiner weiteren Kontrolle der Konzentration mehr.

Im übrigen nimmt man die Carbonylierung wie üblich vor, also bei 80-200, vorzugsweise 110-170° C und unter einem Kohlenmonoxiddruck von 60-300, vorzugsweise 120-280 bar.

Die Konzentration des Cobaltkatalysators liegt vorzugsweise zwischen 0,01 und 10, besonders zwischen 0,1 und 2,0 Gew.% Co, bezogen auf die Gesamtmenge der flüssigen Bestandteile des Reaktionsgemisches (Acrylsäureester, Alkohol, Lösungsmittel, Stickstoffbase und Verfahrensprodukte).

Man kann das Cobalt in Form fertiger Carbonylkomplexe wie $Co_2(CO)_8$ oder in Form beliebiger Cobaltverbindungen wie Cobaltacetat, Cobaltacetylacetonat oder Cobaltstearat einsetzen, da sich die aktiven Cobaltkomplexe unter den Reaktionsbedingungen von selbst bilden.

Unter den Promotoren ist in erster Linie Pyridin zu nennen, jedoch kommen auch Alkylpyridine wie die Picoline oder Isochinolin in Betracht.

Pro Mol Cobalt verwendet man vorzugsweise mindestens 2 mol der Base, wobei sich Mengen von 10-40 mol besonders empfehlen. Grössere Mengen, etwa bis zu 400 mol sind möglich, bedingen im allgemeinen jedoch keine Vorteile mehr.

Die Menge des Alkohols soll mindestens äquimolar zur Menge des Acrylsäureesters sein, jedoch empfiehlt es sich, dass der Alkohol während der Reaktion stets im Überschuss zum Acrylsäureester vorliegt; bevorzugt wird hier ein Molverhältnis von 2-10 mol Alkohol pro Mol des Acrylsäureesters.

Das erfindungsgemässe Verfahren eröffnet einen sehr vorteilhaften Weg zur Herstellung von Butan-1,4-diol durch Oxidation von Propylen zu Acrylsäure und deren Veresterung zu Acrylestern, Carbonylierung der erhaltenen Acrylsäureester zusammen mit einem Alkohol und Hydrierung der Bernsteinsäurediester auf an sich bekannte Weise zum Butandiol und den Alkoholen.

Da das Verfahren im Hinblick auf die Butandiolsynthese die grösste technische Bedeutung erwarten lässt, wird man in der Regel vom einfachsten und billigsten Acrylsäureester, nämlich Methylacrylat, und vom einfachsten und billigsten Alkohol, nämlich Methanol, ausgehen.

Andererseits ist das Carbonylierungsverfahren hierauf nicht beschränkt und lässt sich prinzipiell mit Erfolg auch auf die Umsetzung anderer Acrylsäureester und anderer Alkohole anwenden.

Als Alkoholkomponente in den Acrylsäureestern kommen daher allgemein aliphatische, cycloaliphatische und araliphatische Alkohole in Betracht, die sich unter den Carbonylierungsbedingungen inert verhalten. Bevorzugt werden $C_1$-$C_8$-Alkanole, besonders $C_1$-$C_3$-Alkanole und darunter, wie bereits erwähnt, vor allem Methanol.

Für die Alkohole, welche in die Carbonylierungsreaktion eingehen, gilt das gleiche.

Sollen die Alkoholreste aus den Bernsteinsäurediestern wieder abgespalten werden, so ist es zweckmässig, den gleichen Alkohol für die Carbonylierung einzusetzen, wie er im Acrylsäureester in gebundener Form vorliegt.

Zur Unterbindung unerwünschter Polymerisationen empfiehlt es sich, das erfindungsgemässe Carbonylierungsverfahren in Gegenwart wirksamer Mengen von Polymerisationsinhibitoren wie Hydrochinon oder N,N'-Di-β-naphthyl-p-phenylendiamin vorzunehmen.

Mit Hilfe des erfindungsgemässen Verfahrens lassen sich bei praktisch quantitativem Umsatz Ausbeuten an den Bernsteinsäurediestern von über 90% erzielen.

*Beispiel 1:*

Eine Mischung aus 180 g (5,6 mol) Methanol, 15 g (0,044 mol) Dicobaltoctacarbonyl, 23,7 g (0,3 mol) Pyridin und 200 g Toluol (= rd. 50 Gew.% der flüssigen Bestandteile) wurde bei 130° C unter einem CO-Druck von 120-130 bar im Laufe von 3 Stunden kontinuierlich mit 258 g (3,0 mol) Methylacrylat versetzt.

Wie durch GC-Analyse von Proben, die im Abstand von 30 min gezogen wurden, festgestellt wurde, stieg die Konzentration des Acrylsäureesters, bezogen auf die flüssigen Bestandteile des Reaktionsgemisches von 0,3 auf 2,8 Gew.% an.

Zur Vervollständigung des Umsatzes wurde das Reaktionsgemisch nach beendetem Zulauf des Methylacrylates noch weitere 3 h bei 130° C und unter dem CO-Druck von 120-130 bar behalten. Die übliche destillative Aufarbeitung des Reaktionsgemisches lieferte das Dimethylsuccinat in einer Ausbeute von 94,2%.

Mit den gleichen Mengen der Einsatzstoffe sowie bei gleicher Temperatur und gleichem Druck, jedoch in Gegenwart der Gesamtmenge des Methylacrylates von Anfang an (Anfangskonzentration rd. 39 Gew.%) wurde nur eine Dimethylsuccinat-Ausbeute von 82,6% erzielt.

Wurde das Methylacrylat zwar im Laufe von 3 h allmählich zur Reaktion gebracht, jedoch kein Lösungsmittel mitverwendet, wurde lediglich eine Dimethylsuccinat-Ausbeute von 76,5% erzielt.

Bei Vorlage des gesamten Methylacrylates und ohne Mitverwendung des Lösungsmittels, jedoch sonst mit gleichen Mengen der Einsatzstoffe und bei gleicher Temperatur und gleichem Druck betrug die Dimethylsuccinat-Ausbeute nur 75,5%.

*Beispiel 2:*

Auf die erfindungsgemässe Arbeitsweise von Beispiel 1, jedoch bei 150° C und 280 bar, wurde eine Dimethylsuccinat-Ausbeute von 95,1% erzielt. Die Konzentration des Methylacrylates stieg hierbei von 0,3 auf 2,5 Gew.% an, um in der Nachreaktion wieder auf Null abzunehmen.

Ohne Mitverwendung des Toluols und in Gegenwart der Gesamtmenge des Methylacrylates von Anfang an wurde nur eine Dimethylsuccinat-Ausbeute von 81,0% erreicht.

*Beispiel 3:*

Auf die erfindungsgemässe Arbeitsweise von Beispiel 1, jedoch unter Verwendung von n-Butylacrylat als Acrylester und Butanol-1 als Alkohol, wurde eine Dibutylsuccinat-Ausbeute von 91,2% erzielt.

*Beispiel 4:*

Auf die erfindungsgemässe Arbeitsweise von Beispiel 1, jedoch unter Verwendung von Cyclohexylacrylat als Acrylester und Cyclohexanol als Alkohol, wurde eine Dicyclohexylacrylat-Ausbeute von 90,0% erreicht.

*Beispiel 5:*

Auf die erfindungsgemässe Arbeitsweise von Beispiel 1, jedoch mit 270 g Dimethylsuccinat als Lösungsmittel, wurde eine Dimethylsuccinat-Ausbeute, bezogen auf umgesetzten Acrylsäuremethylester, von 94,5% erzielt.

## Patentanspruch

Verfahren zur Herstellung von Bernsteinsäurediestern durch Carbonylierung von Acrylsäureestern in Gegenwart von Alkoholen mittels Cobalt-carbonyl-Komplexen als Katalysatoren und heterocyclischen Stickstoffbasen als Promotoren bei 80-200° C und unter einem Kohlenmonoxiddruck von 60-300 bar, sowie in Gegenwart von mindestens 20 Gew.%, bezogen auf die Menge aller flüssigen Bestandteile des Reaktionsgemisches, einer inerten Flüssigkeit, dadurch gekennzeichnet, dass man hierbei die Konzentration des Acrylsäureesters während des überwiegenden Teils der Reaktionsdauer unter 15 Gew.% hält, ebenfalls bezogen auf die Menge aller flüssigen Bestandteile.

## Claim

A process for the preparation of succinic acid diesters by carbonylation of acrylic acid esters in the presence of an alcohol, using a cobalt carbonyl complex as the catalyst and a heterocyclic nitrogen base as the promoter, at 80-200° C and under a carbon monoxide pressure of 60-300 bar, as well as in the presence of at least 20% by weight, based on the amount of all the liquid constituents of the reaction mixture, of an inert liquid, wherein the concentration of the acrylic acid ester is kept, for the predominant part of the reaction time, at below 15% by weight, again based on the amount of all liquid constituents.

## Revendication

Procédé de préparation de diesters de l'acide succinique par une carbonylation d'esters de l'acide acrylique entre 80 et 200° C sous une pression d'oxyde de carbone de 60 à 300 bars en présence d'alcools, de complexes de cobalt-carbonyle comme catalyseurs et de bases azotées hétérocycliques comme promoteurs, ainsi que d'au moins 20% en poids, par rapport à la quantité totale de tous les ingrédients liquides du mélange réactionnel, d'un liquide inerte, caractérisé en ce que, pendant la majeure partie de la durée de la réaction, la concentration de l'ester de l'acide acrylique est maintenue inférieure à 15% du poids total de tous les ingrédients liquides.